# EUROPÄISCHE PATENTSCHRIFT

(11) **EP 1 771 117 B1**
(45) Veröffentlichungstag und Bekanntmachung des Hinweises auf die Patenterteilung: **14.08.2013**
(21) Anmeldenummer: 05757099.6
(22) Anmeldetag: 22.06.2005
(51) Int. Cl.: A61B 17/32

(54) **MEDIZINISCHE PUMPE**
MEDICAL PUMP
POMPE MEDICALE

(30) Priorität: 30.06.2004 DE 102004031674
(43) Veröffentlichungstag der Anmeldung: 11.04.2007
(73) Patentinhaber: ERBE Elektromedizin GmbH, 72072 Tübingen (DE)
(72) Erfinder: KÜHNER, Ralf, 70567 Stuttgart (DE); HAGG, Martin, 72827 Wannweil (DE); QUECK, Jochen, 72076 Tübingen (DE)
(74) Vertreter: Bohnenberger, Johannes
(86) Internationale Anmeldenummer: PCT/EP2005/006753
(87) Internationale Veröffentlichungsnummer: WO 2006/002815

(56) Entgegenhaltungen:
- WO-A-99/33510
- WO-A-02/095234
- WO-A-03/013645

## Beschreibung

Die Erfindung betrifft eine medizinische Pumpe, insbesondere für die Wasserstrahlchirurgie.

In der Leberchirurgie wird seit einiger Zeit die Wasserstrahlchirurgie genutzt, da dieses Organ wie kein anderes über Gewebestrukturen unterschiedlicher Festigkeit verfügt (Parenchym, Blutgefäße, Gallengänge) und somit der applizierte Wasserstrahl das zu schneidende Gewebe (Parenchym) zwar durchtrennt, die Blutgefäße und Gallengänge jedoch unbeschädigt lässt. Hierzu ist natürlich eine exakte Steuerung des Schneiddruckes notwendig.

Ein weiteres Problem in der Wasserstrahlchirurgie besteht darin, dass eine absolute Sterilität des Schneidmediums (z.B. Ringer-Lösung) gegeben sein muss, da die Flüssigkeit in die denkbar engste und intensivste Verbindung mit dem Körpergewebe gelangt. Darüber hinaus sind natürlich die üblichen Probleme wie hohe Zuverlässigkeit, Einfachheit und kostengünstige Herstellbarkeit zu beachten.

Aus der US 6,216,573 B1 und aus der DE 203 09 616 U1 sind medizinische Pumpen für die Wasserstrahlchirurgie bekannt, die auswechselbare, also zur einmaligen Verwendung ausgebildete Pumpeinheiten aufweisen, die mit Pumpenbetätigungseinrichtungen verbindbar sind. Das Auswechseln der Pumpeinrichtungen ist jedoch bei den bekannten Anordnungen sehr aufwändig. Dadurch nämlich, dass relativ große Kräfte benötigt werden, um einen hohen Druck bei hinreichenden Durchflussmengen zu erzeugen, müssen die Einrichtungen zum Verbinden der Pumpeinrichtung mit den Pumpenbetätigungseinrichtungen sehr stabil ausgebildet sein und die Pumpeinrichtung "fest im Griff" haben. Dokument WO-A-99/33510 zeigt auch ein System für die Wasserstrahlchirurgie und bildet die Basis für Anspruch 1.

Der Erfindung liegt die Aufgabe zu Grunde, eine medizinische Pumpe der eingangs genannten Art dahin gehend weiter zu bilden, dass die Verbindung zwischen der Pumpeinheit und der Pumpenbetätigungseinrichtung verbessert und ihre Bedienbarkeit erleichtert wird.

Diese Aufgabe wird durch eine medizinische Pumpe nach dem Patentanspruch 1 gelöst.

Ein wesentlicher Punkt der Erfindung liegt also darin, dass die Antriebseinrichtung, die an sich zum Hin- und Herschieben der Kolben vorgesehen ist, zusätzlich auch die Halteeinrichtungen und/oder die Kupplungseinrichtungen betätigt bzw. steuert. Auf diese Weise ist eine einfache Bedienbarkeit (nämlich durch die Antriebseinrichtung) gewährleistet, andererseits kann eine hohe Haltekraft ausgeübt werden, da diese beim Öffnen zum Abkoppeln der Pumpeinheit von der Antriebseinrichtung und nicht vom Benutzer aufgebracht wird.

Vorzugsweise umfassen die Halteeinrichtungen und/oder die Kupplungseinrichtungen Schnappverbindungseinrichtungen, die derart ausgebildet sind, dass das Schließen der Halteeinrichtungen und/oder der Kupplungseinrichtungen durch Einschnappen und das Öffnen mittels der Antriebseinrichtungen durch Öffnen der Schnappverbindungseinrichtungen durchführbar ist. Die zum Verbinden der Pumpeinrichtung mit der Antriebseinrichtung notwendige Energie kann mit längeren Wegen bei großer Haltektaft vom Benutzer sehr leicht aufgebracht werden. Andererseits wird dann bei einer entsprechend großen Haltekraft das Öffnen durch die Antriebseinrichtungen bewerkstelligt, so dass nur das Öffnen und nicht auch noch das Schließen durch die Antriebseinrichtungen erfolgen muss. Dies vereinfacht die Anordnung.

Die Schnappverbindungseinrichtungen sind vorzugsweise derart ausgebildet, dass eine zum Schließen aufzubringende Kraft geringer als eine zum Öffnen aufzubringende Kraft ist.

Die Antriebseinrichtung ist vorzugsweise derart ausgebildet, dass in der Pumpenbetätigungseinrichtung angebrachte Rasteinrichtungen der Kupplungseinrichtungen in einem Ruhezustand vor Befestigen der Pumpeinheit an der Pumpenbetätigungseinrichtung derart positioniert sind, dass bei einem Verbinden der Pumpeneinheit mit der Pumpenantriebseinrichtung die Rasteinrichtungen außer Eingriff mit den Kolbenstangen sind und die Kupplungseinrichtungen durch Betätigen der Antriebseinrichtungen schließbart sind. Dies bedeutet, dass der Benutzer keine größeren Manipulationen insbesondere in Bezug auf die Kolben bzw. Kolbenpositionen vornehmen muss, um die Pumpeinheit an der Pumpenbetäügungseüiüchtung anzubringen.

Vorzugsweise sind zwei Kolben mit Kolbenstangen in Zylindern vorgesehen und die Pumpenbetätigungseinrichtung ist zum alternierenden Verschieben der Kolben ausgebildet. Dadurch kann eine erhöhte Pumpleistung sichergestellt werden. Bei dieser Ausführungsform ist die Pumpenbetätigungseinrichtung durch zwei Motoren oder einen Motor mit einem steuerbaren Getriebe vorzugsweise derart steuerbar ausgebildet, dass die Kolben zusätzlich zum alternierenden Betrieb zum Öffnen oder Schließen der Halteeinrichtung und/oder der Kupplungseinrichtungen synchron verschiebbar sind. Während also beim normalen Pumpvorgang die Kolben alternierend betätigt werden, wird zum Öffnen oder Schließen der Halteeinrichtung und/oder der Kupplungseinrichtungen ein anderer Betriebsmodus mit ein und denselben Antriebsaggregaten gewählt, was einen vereinfachten Aufbau mit sich bringt.

Die Antriebseinrichtung umfasst vorzugsweise einen Linearantrieb (bzw. bei zwei Kolben zwei Linearantriebe) mit Spindel und Motor zum steuerbaren Antreiben der Spindel. Über derartige Linearantriebe können sehr exakte und darum die Pumpe mit ihren Kolben/Zylindereinheiten schonende Bewegungen durchgeführt werden.

Die Motorsteuerung ist vorzugsweise derart ausgebildet, dass die Kolben in konstanter Geschwindigkeit verschiebbar sind. Dadurch ergibt sich eine gleichmäßigere Förderung des zu pumpenden Mediums.

Bevorzugte Ausführungsformen der Erfindung ergeben sich aus den Unteransprüchen.

Nachfolgend werden bevorzugte Ausführungsformen der Erfindung anhand von Abbildungen näher erläutert. Hierbei zeigen
- Fig. 1 ein schematisches Blockschaltbild einer Ausführungsform der medizinischen Pumpe,
- Fig. 2 eine perspektivische Explosionsdarstellung einer Ausführungsform der Pumpe,
- Fig. 3 eine perspektivische Darstellung der Pumpenbetätigungseinrichtung mit angekoppelter Pumpeinheit,
- Fig. 4 eine Seitenansicht einer Halteeinrichtung,
- Fig. 5 eine Darstellung ähnlich der nach Fig. 4 jedoch in einem Teil-Schnitt entlang der Linie V-V aus Fig. 6, - Fig. 6 eine Darstellung der Anordnung nach Fig. 4 in Draufsicht,
- Fig. 7-9 Darstellungen entsprechend denen nach den Fig. 4-6 jedoch in geöffnetem Zustand der Halteeinrichtung,
- Fig. 10 eine perspektivische Explosionsdarstellung von Funktionseinheiten der Halteeinrichtung,
- Fig. 11 eine perspektivische Darstellung einer Untereinheit aus Fig. 10,
- Fig. 12 eine Explosionsdarstellung einer Untereinheit aus Fig. 11,
- Fig. 13 eine perspektivische Darstellung von Kupplungseinrichtungen,
- Fig. 14 eine Darstellung der Kupplungseinrichtungen aus Fig. 13 in einer anderen Verschiebeposition,
- Fig. 15 eine Explosionsdarstellung einer Kupplungseinrichtung aus den Fig. 13 oder 14,
- Fig. 16 eine perspektivische Explosionsdarstellung einer Untereinheit der Anordnung nach Fig. 13,
- Fig. 17 eine Draufsicht auf die Anordnung nach Fig. 13 mit angekoppelten Kolbenstangen,
- Fig. 18 eine Vorderansicht der Anordnung nach Fig. 17,
- Fig. 19 eine Ansicht entsprechend der nach Fig. 17 jedoch in einem anderen Betriebszustand,
- Fig. 20 eine Vorderansicht auf die Anordnung nach Fig. 19,
- Fig. 21 einen Schnitt entlang der Linie XXI-XXI aus Fig. 19.

In der nachfolgenden Beschreibung werden für gleiche und gleich wirkende Teile dieselben Bezugsziffern verwendet.

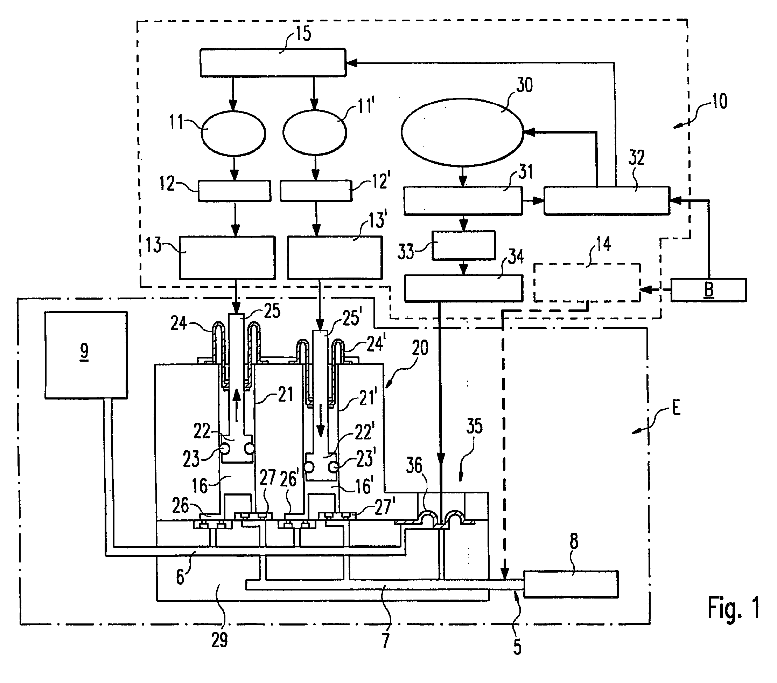

Bei der in Figur 1 dargestellten Ausführungsform der Erfindung ist eine Pumpenbetätigungseinrichtung 10 vorgesehen, welche eine Motorsteuerung 15 zur Steuerung von zwei Motoren 11, 11' umfasst, die über Getriebe 12, 12' und Kupplungseinrichtungen 13, 13' mit Kolbenstangen 25, 25' verbunden sind. Eine Bedienungsperson B kann durch geeignete Schalteinrichtungen (Fußschalter, Fingerschalter) die Motorsteuerung 15 derart betätigen, dass die Motoren 11, 11' über die beschriebene Kette die Kolbenstangen 25, 25' und damit Kolben 22, 22' in Zylindern 21, 21' einer Pumpeneinheit 20 alternierend verschieben, so dass Druckräume 16, 16' der Pumpeneinheit 20 alternierend in ihrem Volumen vergrößert und verkleinert werden.

Zur Abdichtung der Druckräume 16, 16' bzw. der Kolben 22, 22' gegenüber den Zylindern 21, 21' sind an den Kolben 22, 22' Dichtungen 23, 23' vorgesehen. Darüber hinaus sind die Kolbenstangen 25, 25' über Rollmembranen 24, 24', welche einerseits mit den Zylindern 21, 21' und andererseits mit den Kolbenstangen 25, 25' fest verbunden sind, keimdicht abgedichtet. Auf diese Weise können Keime, die sich aus der Umgebungsluft ohne diese Rollmembranen 24, 24' an den Innenwänden der Zylinder 21, 21' absetzen und von den Dichtungen 23, 23' durchgelassen werden, nicht mit dem Arbeitsfluid vermischen bzw. in dieses gelangen.

Mit den Druckräumen 16, 16' verbunden sind Saugventile 26, 26' sowie Druckventile 27, 27'. Die Saugventile 26, 26' stehen über einen Fluideinlass 6 mit einem Vorratsbehälter 9 für das Arbeitsfluid in Verbindung. Die Druckventile 27, 27' stehen über einen Fluidauslass 7 mit einem Druckschlauch 5 in Verbindung, der zu einem Applikator 8 führt. Die Pumpeinheit 20 bildet zusammen mit dem Vorratsbehälter 9 samt seinem Inhalt, dem Druckschlauch 5 und dem Applikator 8 ein Einwegteil E, das nach jeder Operation entsorgt wird, so dass die Gesamtanordnung den allerhöchsten Sterilitätsanforderungen genügt.

Ein Klemmventil 14 ist vorgesehen, über welches (zusätzlich zur Motorsteuerung 15) ein vollständiges Abstellen des Fluidflusses durch die Bedienungsperson B geschehen kann. Die in Figur 1 gezeigte Ausführungsform der Erfindung ist ein Druckregelventil 35, das mittels einer Ventilmembran 36 einen Verbindungskanal zwischen dem Fluidauslass 7 und dem Fluideinlass 6 öffnen und schließen kann. Die Membran 36 wird über eine Stößelstange 34 und eine Feder 33 sowie einen Kraftmesser 31 von einem Stellmotor 30 betätigt. Der Kraftmesser 31 liefert ein kraftproportionales Ausgangssignal an einen Regler 32, über welchen eine Bedienungsperson B einen Maximaldruck vorgeben kann. Statt eines gesonderten Kraftmessers 31 kann auch ein Betätigungsstrom des Stellmotors 30 gemessen werden, der ebenfalls kraftproportional ist.

Durch diese Anordnung ist gewährleistet, dass der Fluiddruck am Applikator 8 exakt eingestellt werden kann. Darüber hinaus werden durch die Kolbenbetätigung auftretende Druckschwankungen durch das Regelventil 35 ausgeglichen. Ein wesentlicher Punkt hierbei liegt darin, dass das Druckregelventil 35 aufgrund seiner Konstruktion mit einer vom Fluiddruck beaufschlagten Membran kraftgesteuert arbeitet und nicht weggesteuert. Dadurch kann bei einem Ankoppeln der Pumpeneinheit 20 an die Pumpenbetätigungseinrichtung 10 auch bei Maßtoleranzen kein Druckeinstellungsfehler auftreten, da es nicht auf die geometrischen Maße (also den Weg), sondern vielmehr auf die Kraft ankommt, mit welcher das Druckregelventil 35 betätigt wird.

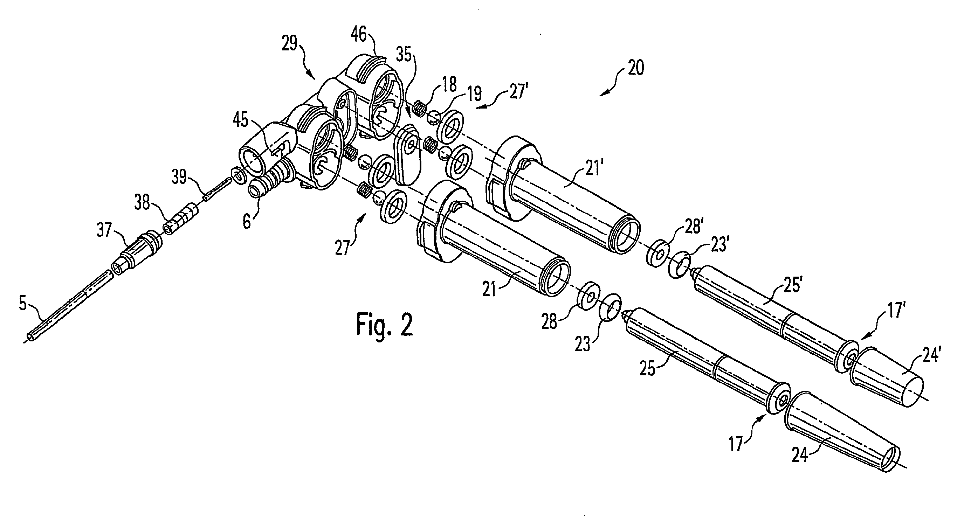

In Figur 2 ist eine konstruktive Ausführungsform der Pumpeinrichtung 20 in einer perspektivischen Explosionsdarstellung gezeigt. Bei dieser Ausführungsform umfassen die Druck- und Saugventile 26/27 Kugeln 19, die über Federn 18 auf Ventilsitze (in der Abbildung nicht sichtbar) gedrückt werden, wie dies im Prinzip bekannt ist.

Der Zylinderkopf 29 weist zwei Abschnitte zum Ankoppeln der Zylinder 21, 21' auf, wobei die Ventile zwischen den Zylindern 21, 21' und dem Zylinderkopf 29 sitzen.

Weiterhin ist aus der Darstellung nach Figur 2 ersichtlich, dass die Kolbenstangen 25, 25' an ihren distalen Enden Koppelvorsprünge 17, 17' aufweisen, über welche eine mechanische Verbindung mit den Kupplungssystemen 13, 13' bewerkstelligt wird.

Die Kolben werden bei dieser Ausführungsform der Erfindung durch proximale Enden der Kolbenstangen 25, 25' mit aufgesetzten Kappen 28 gebildet, welche gleichzeitig die Dichtungen 23, 23' fest auf den Kolbenstangen 25, 25' halten.

Der Druckschlauch 5 wird am Zylinderkopf 29 über einen Überwurfstutzen 37, ein Crimprohr 38 und ein in den Druckschlauch 5 einzusetzendes Innenrohr irreversibel befestigt, wobei nach einem Zusammenstecken (in an sich bekannter Weise) der Überwurfstutzen 37 im Zylinderkopf 29 mittels einer Schnappzunge 45 gehalten wird, die den Überwurfstutzen 37 im Zylinderkopf 29 irreversibel hält.

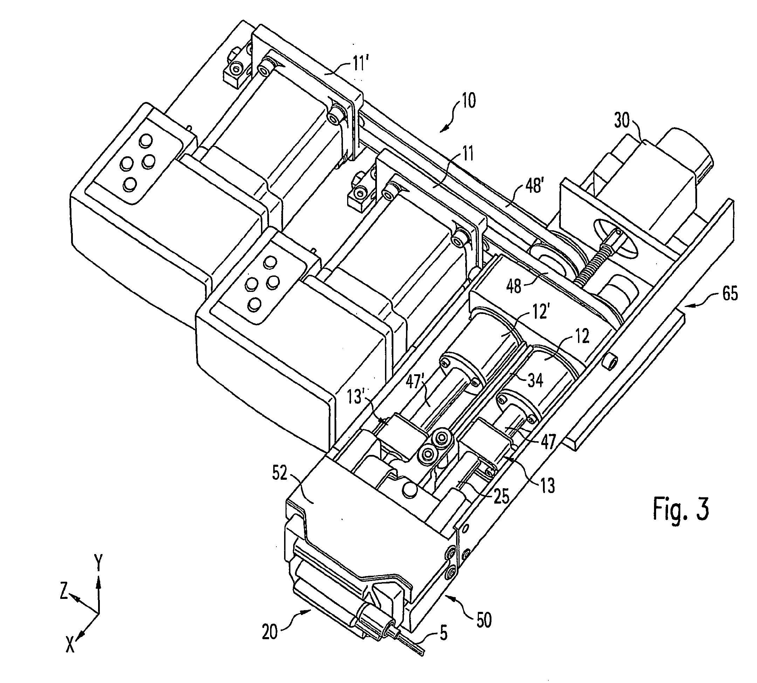

In Fig. 3 ist eine perspektivische Darstellung der Pumpenbetätigungseinrichtung 10 mit einer angekoppelten Pumpeinheit 20 gezeigt. Aus dieser Abbildung geht hervor, dass die Pumpenbetätigungseinrichtung einen Rahmen 65 aufweist, an welchem die Motoren 11, 11' befestigt sind. Diese sind als umsteuerbare Motoren ausgebildet, welche über Zahnriemen 48, 48' und die Getriebe 12, 12' Spindeln 47, 47' antreiben, so dass die Drehbewegung der Motoren 11, 11' in Linearbewegungen umgesetzt wird. An den Spindeln 47, 47' sind die Kupplungen 13, 13' befestigt, an welche die Kolbenstangen 25, 25' ankoppelbar sind. Weiterhin ist aus dieser Abbildung die Anordnung des Stellmotors 30 mit der dazu gehörigen Stößelstange 34 ersichtlich.

Am Rahmen 25 ist weiterhin die Halteeinrichtung 50 angebracht, welche zum Festhalten der Pumpeinheit 20 vorgesehen ist.

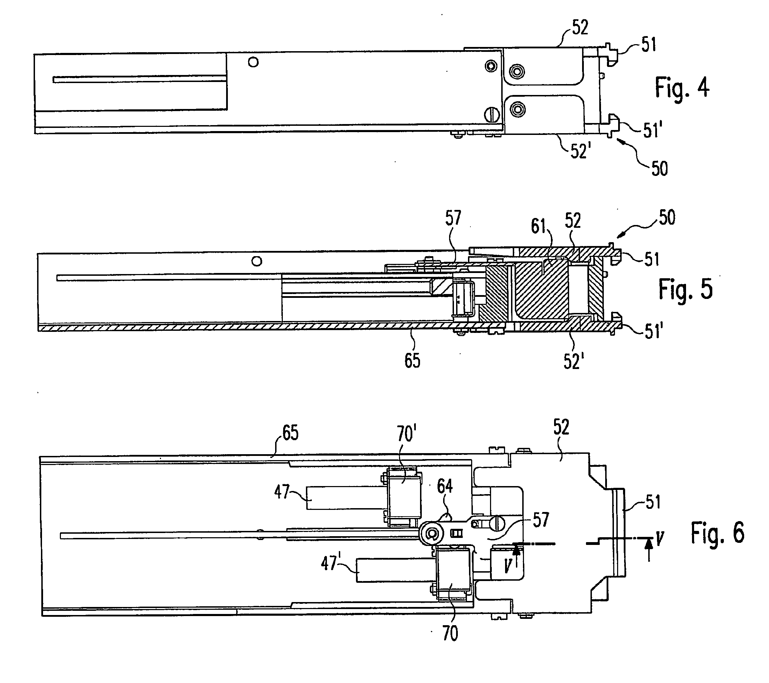

Die Halteeinrichtung 50 wird nachfolgend anhand der Fig. 4 - 12 näher erläutert.

Die Halteeinrichtung 50 umfasst Klauenhalter 52, 52' mit endseitigen Klauen 51, 51', die derart ausgebildet sind, dass sie zum Festhalten der Pumpeinheit 20 mit den dort vorgesehenen Haltevorsprüngen 46 in Eingriff gelangen können.

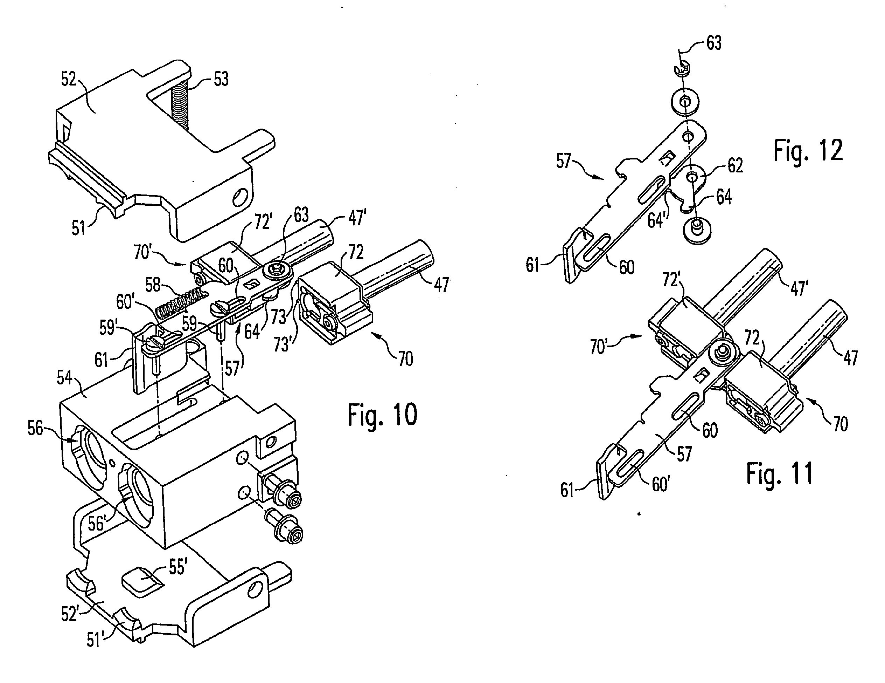

Die Klauenhalter 52, 52' sind - wie in Fig. 4 gezeichnet - über Drehachsen am Rahmen 65 gelagert und mittels Federn 53 (siehe Fig. 10) in Schließstellung (Fig. 4 - 6) vorgespannt. Zum Einsetzen einer Pumpeinheit 20 wird diese so in die Halteeinrichtung 50 gedrückt, dass die Klauen 51, 51' mit vorderseitigen Schrägflächen über die Haltevorsprünge 46 der Pumpeinheit 20 gleiten und aufgezwungen werden. Sobald die Pumpeinheit 20 dann ganz angedrückt ist, schnappen die Klauenhalter 52, 52' zu und die Klauen 51, 51' halten die Pumpeinheit 20 in dieser Position, bis sie wieder auseinander gedrückt werden.

Der Mechanismus zum Öffnen der Halteeinrichtung 50 bzw. der Klauen 51, 51' wird nachfolgend anhand der Fig. 10 - 12 erläutert.

Die Halteeinrichtung 50 umfasst einen Halteblock 54, der an seiner Vorderseite Zylinderaufnahmen 56, 56' aufweist, welche den Hinterseiten der Zylinder 21, 21' der Pumpeinheit 20 entsprechen. Die Passform ist aus einem Vergleich der Fig. 2 und 10 leicht ersichtlich.

An der Zylinderaufnahme 56 ist ein Öffnungsschieber 57 über Befestigungsschtauben 59, 59' befestigt, wobei der Öffnungsschieber 57 Langlöcher 60, 60' aufweist, so dass er vor- und zurückverschiebbar ist. Mittels einer Feder 58 wird der Öffnungsschieber 52 nach hinten, also fort von der Pumpeinheit 20 gedrückt.

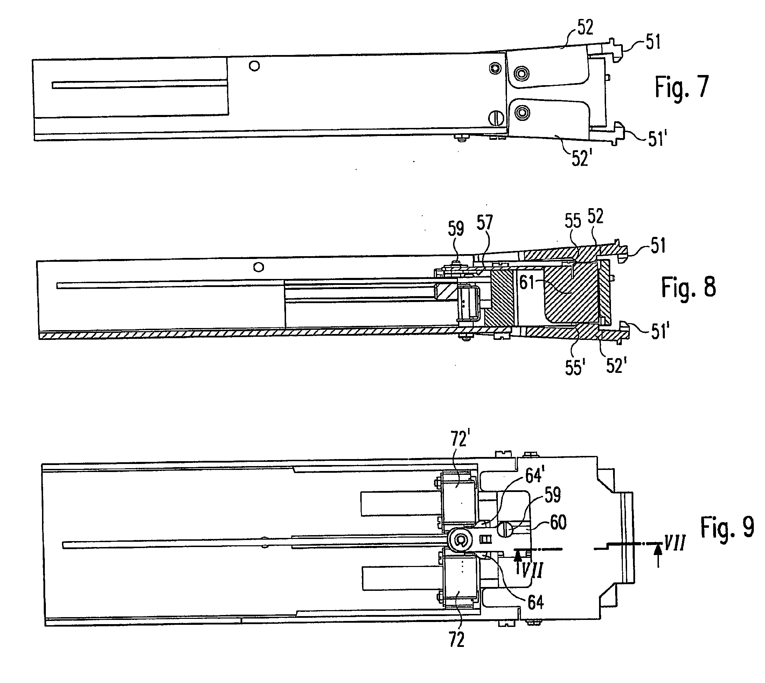

Am Öffnungsschieber 57 ist über ein Kipphebellager 63 ein Kipphebel 52 hin- und herverschwenkbar angebracht, der symmetrisch angeordnete Zungen 64, 64' trägt. An seinem Vorderende, welches der Pumpeinheit 20 zugewandt ist, trägt der Öffnungsschieber 57 eine Öffnungszunge 61. Die Öffnungszunge 61 weist nun eine Höhe auf, welche dem Abstand zwischen den Innenflächen der Klauenhalter 52, 52' entspricht. An diesen Innenflächen der Klauenhaltet 52, 52' sind Öffnungsrampen 55, 55' im Verschiebeweg der Öffnungszunge 61 derart angebracht, dass die Öffnungszunge 61 beim Auftreffen auf die Öffnungsrampen 55, 55' und beim weiteren Verschieben in Richtung auf die Pumpeinheit 50 die Klauenhalter 52, 52' auseinander drückt, so dass sie von der in den Fig. 4 - 6 gezeigten Position übergehen in die in den Fig. 7 - 9 gezeigte Position. In dieser Position (gemäß Fig. 7 - 9) kommen die Klauen 51, 51' außer Eingriff mit den Haltevorsprüngen 46 an der Pumpeinheit 20 und geben diese somit frei. Das Verschieben des Öffnungsschiebers 57 geschieht nun wie nachfolgend beschrieben.

Bei einer "normalen" Betätigung der Pumpeinheit 20 werden die Spindeln 47, 47' alternierend hin- und herbewegt, so dass sie in einer Endposition einer Spindel 47 bzw. 47' die in den Fig. 6 oder 10 gezeigten Positionen einnehmen. Bei diesen Bewegungen werden Halteblöcke 72 und 72' von Kolbenhaltern 70, 70' an den Enden der Spindeln 47, 47' so an den Zungen 64, 64' vorbei bewegt, dass der Kipphebel 62 entweder in einer Richtung entgegen dem Uhrzeigersinn verkippt wird, wie dies in den Fig. 6 oder 10 gezeigt ist oder in die andere Richtung, bei welcher die Halteblöcke 72, 72' in der umgekehrt -vorgezogenen bzw. zurückgezogene Position sind. Diese alternierenden Bewegungen der Kolbenhalter 70, 70' bzw. der Halteblöcke 72, 72' können also zur Betätigung der Pumpe ohne Verschieben des Öffnungsschiebers 57 in Richtung auf die Pumpeinheit 20 durchgeführt werden.

Dann aber, wenn die Spindeln 47, 47' derart angetrieben werden, dass beide Kolbenhalter 70, 70' bzw. Haltblöcke 72, 72' nebeneinander laufen, kann beim Vorschieben (in Richtung auf die Pumpeinheit 20) der Kipphebel 62 nicht ausweichen, so dass beide Halteblöcke 72, 72' mit beiden Haltezungen 64, 64' gleichzeitig in Eingriff gelangen. Durch diesen Eingriff wird nun bei einem weiteren Vorbewegen der Kolbenhalter 70, 70' der Öffnungsschieber 57 entgegen der Kraft der Feder 58 in Richtung auf die Pumpeinheit 50 in seinen Langlöchern 60, 60' verschoben, so dass die Öffnungszunge 61 über die Öffnungsrampen 55, 55' gleitet und damit die Klauenhalter 52, 52' auseinander zwängt. Dadurch wird der Eingriff der Klauen 51, 51' gegenüber den Haltevorsprüngen 46 an der Pumpeinheit 20 gelöst. Dieses Öffnen der Halteeinrichtung 50 geschieht also ausschließlich über die Motoren 11, 11' und deren entsprechende Steuerung durch die Motorsteuerung 15.

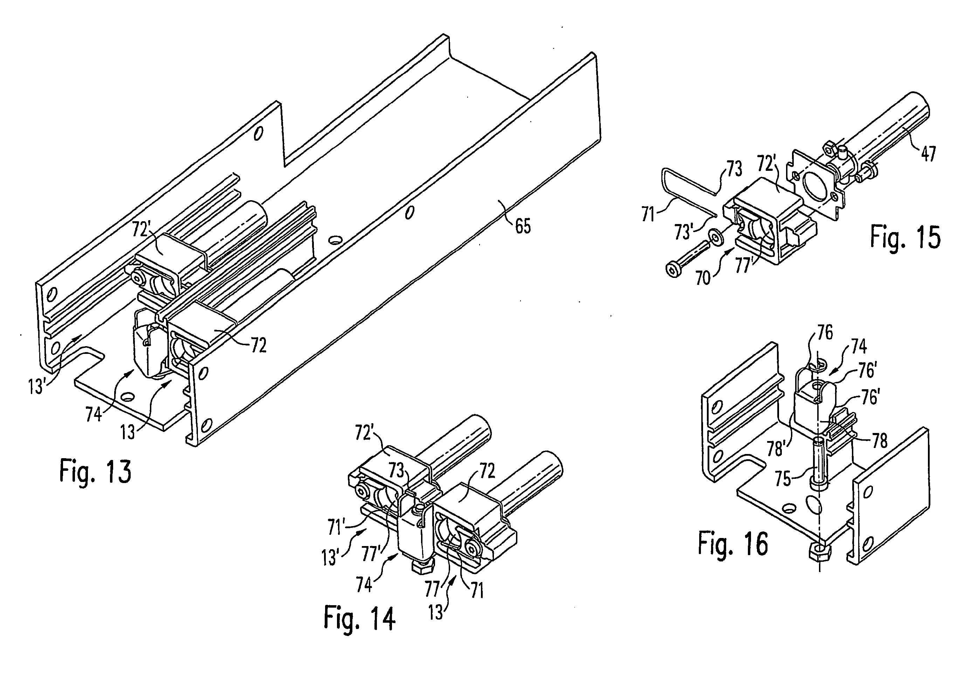

Nachfolgend wird die Wirkung bzw. Betätigung der Kupplungssysteme 13, 13' näher beschrieben, mit welcher die Kolbenstangen 25, 25' über ihre Koppelvorsprünge 17, 17' an die Kolbenhalter 70, 70' angekoppelt werden. Hierzu wird auf die Fig. 13 - 21 verwiesen.

Die Halteblöcke 72, 72' sind gemäß Fig. 15 an die Spindeln 47, 47' angeschraubt und weisen Einsetzöffnungen 77, 77' auf, in welche die Kolbenstangen 25, 25' mit ihren Koppelvorsprüngen 17, 17' eingesetzt werden können. An den Halteblöcken 72, 72' sind Federn 71, 71' so befestigt, dass Federenden 73, 73' in die Einsetzöffnungen 77, 77' ragen. Der Abstand der Federenden 73, 73' ist derart, dass die Kolbenstangen 25, 25' mit ihren Koppelvorsprüngen 17, 17' in die Einsetzöffnungen 77, 77' eingesetzt werden können und dabei die Federenden 73, 73' auseinander zwingen, bis sie hinter den Koppelvorsprüngen 17, 17' zusammenschnappen. Hierfür sind die Koppelvorsprünge 17, 17' an ihren Enden konisch ausgebildet. Nach dem Einsetzen der Koppelvorsprünge 17, 17' in die Kolbenhalter 70, 70' sind die Kolbenstangen 25, 25' schub- und zugfest mit den Kolbenhaltern 70 verbunden.

Zwischen den Verschiebewegen der Kolbenhalter 70, 70' bzw. der Halteblöcke 72, 72' ist auf einem Schwenklager 75 ein Spreizhebel 74 verschwenkbar angebracht, der an seiner Ober- und Unterseite und zwar an dem der Pumpeinheit 20 abgewandten Seite Spreizflächen 76, 76' aufweist. Am anderen, also der Pumpeinheit 20 zugewandten Ende sind Schwenkkanten 78, 78' am Spreizhebel 74 vorgesehen.

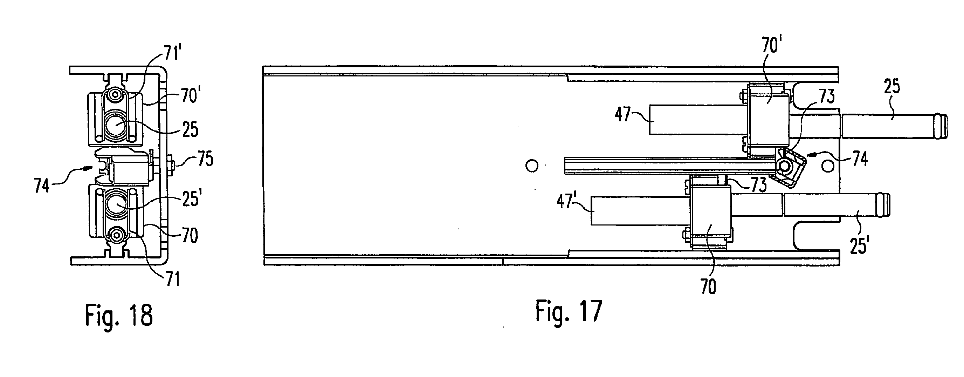

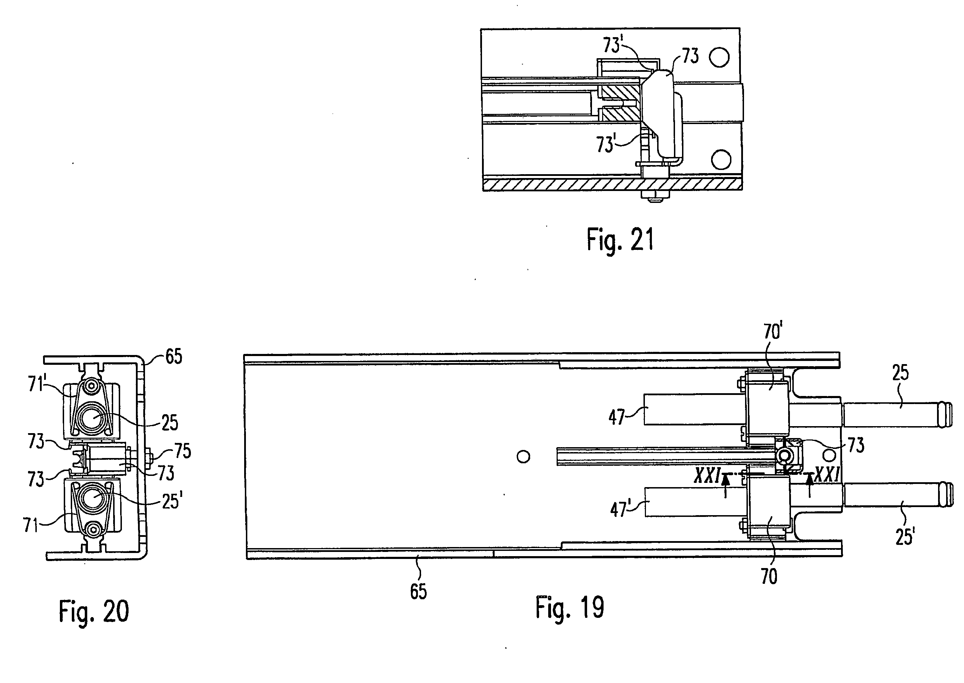

Die Anordnung und Dimensionierung des Spreizhebels 74 mit seinen Spreizflächen 76, 76' und Schwenkkanten 78, 78' ist nun derart getroffen, dass bei einer alternierenden Bewegung der Halteblöcke 72, 72' bzw. Kolbenhalter 70, 70', wie sie in den Fig. 13 und 17 gezeigt ist, der Spreizhebel 74 entweder nach der einen oder nach der anderen Seite verkippt wird, je nachdem, welcher der Kolbenhalter 70, 70' bzw. Halteblöcke 72, 72' auf seinem Weg in Richtung auf die Pumpeinheit 20 an ihm vorbeigleitet. Durch dieses Verschwenken werden die Spreizflächen 76, 76' so verschwenkt, dass sie nicht in Eingriff mit den Federenden 73, 73' des vorbeigleitenden Kolbenhalters 70 bzw. 70' gelangen können. Dann aber, wenn die beiden Kolbenhalter 70, 70' bzw. Halteblöcke 72, 72' parallel nebeneinander in Richtung auf die Pumpeinheit 20 verschoben werden (siehe Fig. 14 und 19 - 21) gelangen die Spreizflächen 76 bzw. 76' mit den Federenden 73, 73' in Eingriff (siehe insbesondere Fig. 20 und 21), so dass diese auf den (angeschrägten) Spreizflächen 76 bzw. 76' entlang gleiten und auseinandergezwängt werden. Durch dieses Auseinandetzwängen werden dann die zuvor an ihren Koppelvorsprüngen 17, 17' festgehaltenen Kolbenstangen 25, 25' (siehe Haltposition gemäß Fig. 18) freigegeben, wie dies in Fig. 20 gezeigt ist. Nachdem bei eben derselben gleichzeitigen und parallelen Bewegung der Kolbenhalter 70, 70' bzw. Halteblöcke 72, 72' auch die Halteeinrichtung 50 geöffnet bzw. die Klauen 51, 51' auseinandergezwängt und damit deren Eingriff mit den Haltevorsprüngen 46 der Pumpeinheit 20 aufgehoben wird, kann die Pumpeinheit bei dem parallelen Verschieben der Kolbenhalter 70, 70' bis zu ihrer vorderen, der Pumpeinheit 20 zugewandten Position ohne Überwindung von Kraft durch den Benutzer entfernt werden.

Die Motorsteuerung 15 ist weiterhin derart ausgebildet, dass nach Abnehmen einer Pumpeinheit 20 von der Pumpenbetätigungseinrichtung 10 die beiden Spindeln 47, 47' die Kolbenhalter 70, 70' zurückziehen. Setzt also der Benutzer eine Pumpeinheit 20 in die Pumpenbetätigungseinrichtung 10 ein, so muss er lediglich die zum Öffnen der Halteeinrichtung 50 notwendige Kraft überwinden. Die Kolbenstangen 25, 25' ragen dann mit ihren Koppelvorsprüngen 17, 17' durch die Zylinderaufnahmen 56, 56' in die Pumpenbetätigungseinrichtung 10 hinein. Der Benutzer kann nun die Motorsteuerung 15 derart ansteuern, dass diese in einem "Kopplungsmodus" die Kolbenhalter 70, 70' in Richtung auf die Pumpeinheit 20 zu bewegt, bis die Koppelvorsprünge 17 bzw. 17' die Federenden 73, 73' auseinander drücken und einschnappen. Dieser Einschnappvorgang wird getrennt für die beiden Koppelvorsprünge 17 bzw. 17' nacheinander vorgenommen, so dass der Spreizhebel 74 die Federn 71, 71' nicht öffnet.

Aus obiger Beschreibung geht hervor, dass ein wesentlicher Punkt der Erfindung darin liegt, dass das Ankoppeln der Pumpeinheit 20 an die Pumpenbetätigungseinrichtung 10 teilweise und das Abkoppeln vollständig über den Antrieb erfolgt, der eigentlich zum Betätigen der Pumpe selbst vorgesehen ist. Gesonderte Antriebseinrichtungen sind hierzu also nicht notwendig.

### Bezugszeichenliste

- E: Einwegteil
- B: Bedienungsperson
- 5: Druckschlauch
- 6: Fluideinlass
- 7: Fluidauslass
- 8: Applikator
- 9: Vorratsbehälter
- 10: Pumpenbetätigungseinrichtung
- 11, 11': Motor
- 12, 12': Getriebe
- 13, 13': Kupplungssystem
- 14: Klemmventil
- 15: Motorsteuerung
- 16, 16': Druckraum
- 17, 17': Koppelvorsprung
- 18: Feder
- 19: Kugel
- 20: Pumpeinheit
- 21, 21': Zylinder
- 22, 22': Kolben
- 23, 23': Dichtung
- 24, 24': Rollmembran
- 25, 25': Kolbenstange
- 26, 26': Saugventil
- 27, 27': Druckventil
- 28: Kappe
- 29: Zylinderkopf
- 30: Stellmotor
- 31: Kraftmesser
- 32: Regler
- 33: Feder
- 34: Stößelstange
- 35: Druckregelventil
- 36: Ventil-Membran
- 37: Überwurfstutzen
- 38: Crimprohr
- 39: Innenrohr
- 45: Schnappzunge
- 46: Haltevorsprünge
- 47, 47': Spindel
- 48, 48': Zahnriemen
- 50: Halteeinrichtung
- 51, 51': Klauen
- 52, 52': Klauenhalter
- 53: Druckfeder
- 54: Halteblock
- 55, 55': Öffnungsrampe
- 56, 56': Zylinderaufnahme
- 57: Öffnungsschieber
- 58: Feder
- 59, 59': Befestigungsschraube
- 60, 60': Langloch
- 61: Öffnungszunge
- 62: Kipphebel
- 63: Kipphebellager
- 64, 64': Zungen
- 65: Rahmen
- 70: Kolbenhalter/Rasteinrichtungen
- 71, 71': Feder
- 72, 72': Halteblock
- 73, 73': Federenden
- 74: Spreizhebel
- 75: Lager
- 76, 76': Spreizflächen
- 77, 77': Einsetzöffnung
- 78, 78': Schwenkkanten

## Patentansprüche

1. Medizinische Pumpe insbesondere für die Wasserstrahlchirurgie, umfassend
- eine Pumpeinheit (20), die als Einwegartikel aufgebaut ist, mit zwei Kolben (22, 22') mit Kolbenstangen (25, 25') zum Verschieben der Kolben (22, 22') in zugeordneten Zylindern (21, 21');
- eine Pumpenbetätigungseinrichtung (50) mit mindestens einer Antriebseinrichtung (11, 12) und einer Motorsteuerung (15) zum Betätigen der Pumpeinheit (20) durch Verschieben der Kolbenstangen (25, 25');
- öffen- und schließbare Halteeinrichtungen (46, 50) zum reversiblen Befestigen der Pumpeinheit (20) an der Pumpenbetätigungseinrichtung (10);
- öffen- und schließbare Kupplungseinrichtungen (13, 17, 70) zum reversiblen Verbinden der Kolbenstangen (25, 25') mit der Antriebseinrichtung (11, 12),
**dadurch gekennzeichnet, dass**
die Antriebseinrichtung (11, 12) zum Öffnen und/oder Schließen der Halteeinrichtungen (46, 50) und/oder der Kupplungseinrichtung (13, 17) steuerbar ausgebildet ist, wobei
die Pumpenbetätigungseinrichtung (10) zum alternierenden Verschieben der Kolben (22) ausgebildet ist, und
die Pumpenbetätigungseinrichtung (10) durch zwei Motoren (11, 11') oder einen Motor mit Getriebe derart steuerbar ausgebildet ist, dass die Kolben (22, 22') zusätzlich zum alternierenden Betrieb zum Öffnen oder Schließen der Halteeinrichtung (46, 50) und/oder der Kupplungseinrichtungen (13) synchron verschiebbar sind.

2. Medizinische Pumpe nach Anspruch 1,
**dadurch gekennzeichnet, dass**
die Halteeinrichtungen (49, 50) und/oder die Kupplungseinrichtungen (13, 17) Schnappverbindungseinrichtungen (49, 51; 17, 70) umfassen, die derart ausgebildet sind, dass das Schließen der Halteeinrichtungen (49, 50) und/oder der Kupplungseinrichtung (13, 17, 70) durch Einschnappen und das Öffnen mittels der Antriebseinrichtung (11, 12) durch Öffnen der Schnappverbindungseinrichtungen (49, 51; 17, 70) durchführbar ist.

3. Medizinische Pumpe nach einem der vorherigen Ansprüche, insbesondere nach Anspruch 2,
**dadurch gekennzeichnet, dass**
die Schnappverbindungseinrichtung (49, 51; 17, 70) derart ausgebildet sind, dass eine zum Schließen aufzubringende Kraft geringer als eine zum Öffnen aufzubringende Kraft ist.

4. Medizinische Pumpe nach einem der vorhergehenden Ansprüche, insbesondere nach einem der Ansprüche 2 oder 3,
**dadurch gekennzeichnet, dass**
die Antriebseinrichtung (11, 12) derart ausgebildet ist, dass in der Pumpenbetätigungseinrichtung (10) angebrachte Rasteinrichtungen (70) der Kupplungseinrichtungen (13, 17, 70) in einem Ruhezustand vor Befestigen der Pumpeinheit (20) an der Pumpenbetätigungseinrichtung (10) derart positioniert sind, dass bei einem Verbinden der Pumpeinheit (20) mit der Pumpenantriebseinrichtung (10) die Rasteinrichtungen (70) außer Eingriff mit den Kolbenstangen (25) sind und die Kupplungseinrichtungen (13) durch Betätigen der Antriebseinrichtungen (11, 12) schließbar sind.

5. Medizinische Pumpe nach einem der vorhergehenden Ansprüche,
**dadurch gekennzeichnet, dass**
die Antriebseinrichtung (11, 12) einen Linearantrieb mit mindestens einer Spindel (47) und mindestens einem Motor (11) zum steuerbaren Antrieb der Spindel (47) umfasst.

6. Medizinische Pumpe nach einem der vorhergehenden Ansprüche,
**dadurch gekennzeichnet, dass**
die Motorsteuerung (15) derart ausgebildet ist, dass die Kolben (22) in konstanter Geschwindigkeit verschiebbar sind.

## Claims

1. Medical pump, in particular for water jet surgery, comprising
- a pump unit (20), which is designed as a disposable article and has two pistons (22, 22'), with piston rods (25, 25') for displacing the pistons (22, 22') in allocated cylinders (21, 21');
- a pump-actuating device (10) with at least one drive device (11, 12) and a motor control (15) for the actuation of the pump unit (20) by displacement of the piston rods (25, 25');
- holding devices (46, 50), which can be opened and closed for reversibly securing the pump unit (20) on the pump-actuating device (10);
- coupling devices (13, 17, 70), which can be opened and closed for reversibly connecting the piston rods (25, 25') to the drive device (11, 12),
**characterized in that**
the drive device (11, 12) is designed such that it can be controlled for opening and/or closing the holding devices (46, 50) and/or the coupling device (13, 17), wherein
the pump-actuating device (10) is designed for alternating displacement of the pistons (22), and
the pump-actuating device (10) is designed to be controllable by two motors (11, 11') or one motor with gear, in such a way that the pistons (22, 22'), in addition to the alternating operation, are displaceable in synchrony for opening or closing the holding device (46, 50) and/or the coupling devices (13).

2. Medical pump according to Claim 1, **characterized in that** the holding devices (49, 50) and/or the coupling devices (13, 17) comprise snap connection devices (49, 51; 17, 70), which are designed in such a way that the closing of the holding devices (49, 50) and/or of the coupling device (13, 17, 70) can be performed by snap-fit engagement, and the opening by means of the drive device (11, 12) can be performed by opening of the snap connection devices (49, 51; 17, 70).

3. Medical pump according to one of the preceding claims, in particular according to Claim 2, **characterized in that** the snap connection devices (49, 51; 17, 70) are designed in such a way that a force that has to be applied to close them is lower than a force that has to be applied to open them.

4. Medical pump according to one of the preceding claims, in particular according to one of Claims 2 and 3, **characterized in that** the drive device (11, 12) is designed in such a way that latching devices (70) of the coupling devices (13, 17, 70), mounted in the pump-actuating device (10), are positioned, in a rest state prior to securing of the pump unit (20) on the pump-actuating device (10), in such a way that when the pump unit (20) is connected to the pump-actuating device (10), the latching devices (70) are disengaged from the piston rods (25), and the coupling devices (13) can be closed by actuation of the drive devices (11, 12).

5. Medical pump according to one of the preceding claims, **characterized in that** the drive device (11, 12) comprises a linear drive with at least one spindle (47) and with at least one motor (11) for controllably driving the spindle (47).

6. Medical pump according to one of the preceding claims, **characterized in that** the motor control (15) is designed in such a way that the pistons (22) can be displaced at constant speed.

## Revendications

1. Pompe médicale, en particulier pour la chirurgie à jet d'eau, comprenant :
- une unité de pompe (20) qui est réalisée sous forme d'article à usage unique, comprenant deux pistons (22, 22') avec des tiges de pistons (25, 25') pour déplacer les pistons (22, 22') dans des cylindres associés (21, 21') ;
- un dispositif d'actionnement de pompe (10) comprenant au moins un dispositif d'entraînement (11, 12) et une commande par moteur (15) pour l'actionnement de l'unité de pompe (20) par déplacement des tiges de pistons (25, 25') ;
- des dispositifs de retenue pouvant être ouverts et fermés (46, 50) pour la fixation réversible de l'unité de pompe (20) au dispositif d'actionnement de pompe (10) ;
- des dispositifs d'accouplement pouvant être ouverts et fermés (13, 17, 70) pour la connexion réversible des tiges de pistons (25, 25') au dispositif d'entraînement (11, 12), **caractérisée en ce que**
le dispositif d'entraînement (11, 12) est réalisé de manière à pouvoir être commandé pour ouvrir et/ou fermer les dispositifs de retenue (46, 50) et/ou le dispositif d'accouplement (13, 17), le dispositif d'actionnement de pompe (10) étant réalisé pour déplacer les pistons suivant un mouvement alternatif, et
le dispositif d'actionnement de pompe (10) étant réalisé de manière à pouvoir être commandé par deux moteurs (11, 11') ou un moteur avec une transmission, de telle sorte que les pistons (22, 22') puissent être déplacés de manière synchrone en plus du fonctionnement alternatif pour l'ouverture ou la fermeture du dispositif de retenue (46, 50) et/ou des dispositifs d'accouplement (13).

2. Pompe médicale selon la revendication 1,
**caractérisée en ce que**
les dispositifs de retenue (49, 50) et/ou les dispositifs d'accouplement (13, 17) comprennent des dispositifs de connexion par encliquetage (49, 51 ; 17, 70), qui sont réalisés de telle sorte que la fermeture des dispositifs de retenue (49, 50) et/ou du dispositif d'accouplement (13, 17, 70) puisse être effectuée par encliquetage et que l'ouverture puisse être effectuée au moyen du dispositif d'entraînement (11, 12) par ouverture des dispositifs de connexion par encliquetage (49, 51 ; 17, 70).

3. Pompe médicale selon l'une quelconque des revendications précédentes, en particulier selon la revendication 2,
**caractérisée en ce que**
les dispositifs de connexion par encliquetage (49, 51 ; 17, 70) sont réalisés de telle sorte qu'une force devant être appliquée pour la fermeture soit inférieure à une force devant être appliquée pour l'ouverture.

4. Pompe médicale selon l'une quelconque des revendications précédentes, en particulier selon l'une quelconque des revendications 2 ou 3,
**caractérisée en ce que**
le dispositif d'entraînement (11, 12) est réalisé de telle sorte que des dispositifs d'encliquetage (70) des dispositifs d'accouplement (13, 17, 70) montés dans le dispositif d'actionnement de pompe (10) soient positionnés au niveau du dispositif d'actionnement de pompe (10) dans un état de repos avant la fixation de l'unité de pompe (20) de telle sorte que lors d'une connexion de l'unité de pompe (20) au dispositif d'actionnement de pompe (10), les dispositifs d'encliquetage (70) soient désengagés des tiges de pistons (25) et que les dispositifs d'accouplement (13) puissent être fermés par l'actionnement des dispositifs d'entraînement (11, 12).

5. Pompe médicale selon l'une quelconque des revendications précédentes,
**caractérisée en ce que**
le dispositif d'entraînement (11, 12) comprend un entraînement linéaire avec au moins une broche (47) et au moins un moteur (11) pour l'entraînement commandable de la broche (47).

6. Pompe médicale selon l'une quelconque des revendications précédentes,
**caractérisée en ce que**
la commande par moteur (15) est réalisée de telle sorte que les pistons (22) puissent être déplacés à une vitesse constante.
